(19) Europäisches Patentamt / European Patent Office / Office européen des brevets

(11) **EP 2 853 888 A2**

(12) **EUROPEAN PATENT APPLICATION**

(43) Date of publication:
01.04.2015 Bulletin 2015/14

(51) Int Cl.:
*G01N 27/00* (2006.01)   *G01N 21/33* (2006.01)

(21) Application number: 14183418.4

(22) Date of filing: 03.09.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 06.09.2013 US 201314020671

(71) Applicant: **Watkins Manufacturing Corporation**
**Vista, California 92081 (US)**

(72) Inventor: **Brondum, Klaus**
**Ann Arbor, MI Michigan 48104 (US)**

(74) Representative: **Croston, David**
**Withers & Rogers LLP**
**4 More London Riverside**
**London**
**SE1 2AU (GB)**

(54) **Measuring method for chlorine and pH**

(57)    A method of measuring the level of chlorine in a salt water solution and the pH of that solution comprises measuring the UV absorption of a first sample of the first solution to generate a first absorption value, subjecting the solution to electrolysis to generate a catholyte, measuring the UV absorption of the catholyte to generate a second absorption value, and then determining the level of chlorine in the solution and the pH of the solution using the first and second absorption values.

**FIG. 1**

EP 2 853 888 A2

**Description**

BACKGROUND OF THE INVENTION

Field of Invention

The subject disclosure relates to chlorine and pH sensing in fluid solutions and more particularly to a chlorine concentration detection and pH determination system for analyzing chlorine concentration and/or pH in spa or pool water.

RELATED ART

In the prior art, various devices for measuring chlorine (hypochlorous acid) concentration in water are known. Such devices include Oxidation Reduction Potential (ORP) sensors, amperometric sensors and Palin N, N-Diethyl-P-Phenylenediamine (DPD) testing apparatus.

SUMMARY

The following is a summary of various aspects and advantages realizable according to various embodiments of the invention. It is provided as an introduction to assist those skilled in the art to more rapidly assimilate the detailed discussion which ensues and does not and is not intended in any way to limit the scope of the claims which are appended hereto in order to particularly point out the invention.

According to an illustrative embodiment, a method of measuring the level of chlorine in a salt water solution and the pH of that solution comprises measuring the UV absorption of a first sample of the first solution to generate a first absorption value, subjecting the solution to electrolysis to generate a catholyte, and measuring the UV absorption of the catholyte to generate a second absorption value. The level of chlorine in the solution and pH may then be determined using the first and second absorption values.

In an illustrative embodiment, the pH of the catholyte is changed such that all chlorine in the catholyte is converted to ClO⁻ prior to measuring the absorption of the catholyte. In one embodiment, the pH of the catholyte is adjusted to be at or above a selected level prior to measuring the absorption thereof

In one embodiment, the level of chlorine is determined using the equation:

$$[Cl_2] = \frac{A_{293}^{alk} \cdot mw_{Cl_2} \cdot 1000}{\varepsilon \cdot l}$$

where $A_{293}$ is the absorption of ClO⁻ at or near 293 nm, and $A_{293}^{alk}$ is the absorption of the catholyte at or near 293 nm after the electrolysis has changed the pH of the catholyte sufficiently so that all chlorine is in hypochlorite form, "$\varepsilon$" is the extinction coefficient for hypochlorite, "$l$" is the length of the absorption path, and $mw_{Cl_2}$ is the molecular weight of chlorine.

In one embodiment, the pH is determined according to the equation:

$$pH = 7.53 + \log \frac{A_{293}}{A_{293}^{alk} - A_{293}}$$

where $A_{293}$ is the absorption of ClO⁻ at or near 293 nm, and $A_{293}^{alk}$ is the absorption of the catholyte at or near 293 nm after the electrolysis has changed the pH of the catholyte sufficiently so that all chlorine is in hypochlorite form.

In one illustrative embodiment, an electrolysis cell is configured to generate separate anolyte and catholyte solutions. An advantage of the cell is that, in one embodiment, it can also generate chlorine. Thus, the cell may perform a dual function of sensing chlorine and pH, as well as generating chlorine for maintaining chlorine level in a spa or pool at specified levels.

BRIEF DESCRIPTION OF THE DRAWINGS

Fig. 1 is a system block diagram illustrating a system for determining chlorine concentration and pH and testing the performance of that system.
Fig. 2 is a schematic cross-sectional view of an illustrative electrolysis cell.
Fig. 3 is a schematic diagram illustrating fluid flow in a cell such as that of Fig. 2.
Fig. 4 is a block diagram of an embodiment of a spectrum analyzer that may be used to measure a percentage of ultra-violet light transmitted through water according to the present disclosure.

DETAILED DESCRIPTION

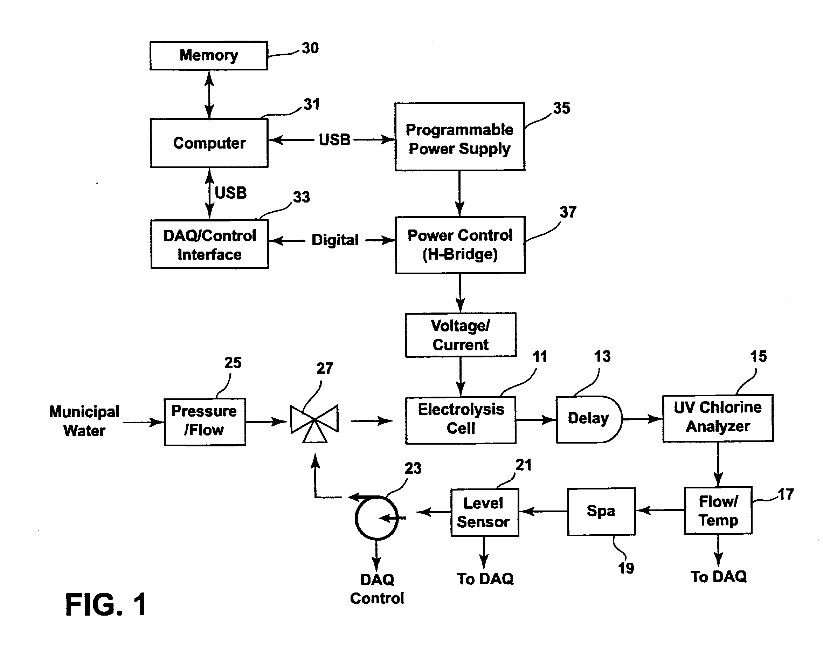

Fig. 1 illustrates a system for determining the chlorine concentration and pH of a solution, in this case spa water, and testing the performance of that system. The system includes an electrolysis cell 11 with separated flows for anolyte and catholyte. The catholyte is flowed out of the electrolysis cell 11 and into a gas liquid separator 13 and then into a UV transmission cell 15. The separator 13 provides a delay which allows the pH and chlorine-hypochlorite equilibrium to settle before UV absorption is measured.

The UV transmission cell 15 may comprise a photodiode with a known emission spectrum to generate a beam which is directed through a water-chlorine solution (e.g. spa water) contained in a cuvette which is, for example, 10 cm in length. The absorption of light is then detected by a photo sensor that is sensitive in the 293 nm region. In practice, it may be difficult to both create a wavelength and detect a wavelength precisely at 293 nm. Thus, in one embodiment, a range is employed, for example, a photodiode that emits light as close to target value as possible i.e., in a range between 280 and 310 nm, and a photodetector that has sensitivity as close to target as possible, for example, between 290-300 nm.

In the system of Fig. 1, water flows from the UV transmission cell 15 to a flow/temp unit 17 and then to the spa 19. The flow/temp unit 17 measures flow rate and temperature, which, in one embodiment, are inputs for chlorine concentration and pH calculation. A flow rate measurement is needed in one embodiment, for example, to control the current applied to the electrolysis system in order to effect a desired or selected change in pH. A flow rate measurement allows calculation of how much current to deliver for what period of time in a system where the saltwater solution is continuously flowing through the cell 11. In a batch system embodiment, a known volume of saltwater is held in the cell 11 and the known volume may be used to calculate the amount of current necessary to create a selected pH change.

Water from the spa 19 next flows to water a level sensor 21 and then to Data Acquisition Control Module (DAQ) unit 23. The DAQ 23 enables comparing measurements determined by the system with reference measurements. From the DAQ Control unit 23, water flows back into the electrolysis cell 11 through a valve 27. The valve 27 also receives a municipal water feed supplied through a pressure/flow measurement unit 25, which is a safety measure to protect the spa heater element (not shown).

Transmission measurements made by the UV transmission cell 15 comprise inputs to chlorine concentration and pH determining algorithms, which are processed by a computing device 31. The computing device 31 may comprise a microprocessor, a microcontroller, or other data processor or computer with suitable tangible memory or computer readable storage medium or media 30 for storage of, for example, non-transitory software, program instructions, program code, data, or information. The electronics further include a DAQ/Control Interface Unit 33, a programmable power supply 35, and a power control (H-Bridge) unit 37, which supplies power (voltage and current) to the electrolysis cell 11.

In one embodiment, transmission data from the UV cell 15 may be fed back to regulate the electrolysis process for optimized sensing and chlorine generation, and the recombined effluent maintains chlorine levels at pre-set levels in the spa bath. In one embodiment, the electrolysis cell 11 may be based on Boron doped diamond (BDD) electrodes and a Nafion separator and operated in switch mode in order to mitigate electrode fouling. The valve 27 enables the water flow from the electrolysis cell 11 to be close looped into the spa bath and also enables the municipal water feed to be supplied to the spa 19 for water level maintenance and sensor calibration routines.

Various other electrode materials may be used in various embodiments, for example, DSA (dimensional stable anode which is noble metal based, for example, alloys of Pd-Ir-Nb (pallidum iridium niobium), or BDD, boron doped diamond, or simply platinum. One of advantage of these materials is that they work in a polarity reversal operation, which is used to mitigate calcium deposits in the cell.

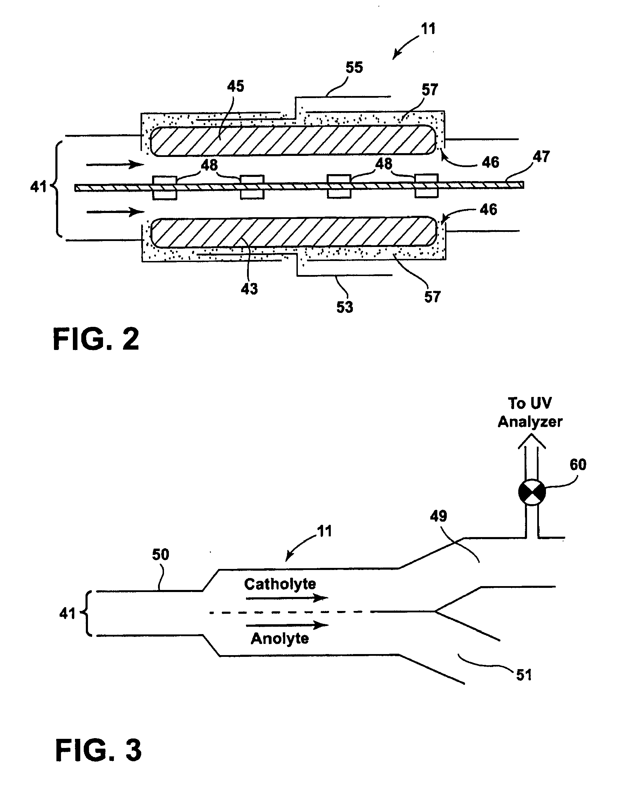

Figs. 2 and 3 schematically show an illustrative embodiment of an electrolysis cell 11. The electrolyte, which in one embodiment is salt water, flows via a tube into the cell 11 through an entrance port 41 (x direction). In one embodiment, the electrolysis occurs in the cell volume (x, y, z) spanned by four peripheral electrodes, namely, two anodes 43 and two cathodes 45. The electrolysis products, now called the anolyte and the catholyte, are kept separate by a separator felt material 47 (e.g. glass felt or Nafion film) and supported by separator support grids, e.g. 48, on both sides. There are two exits 49, 51 from cell 11, which prevents mixing of the anolyte and catholyte. Only one pair of anode-cathodes 43, 45 is shown in Figure 2. In the illustrative embodiments, the second anode-cathode pair is positioned similarly downstream of the first pair 43, 45. Other embodiments may employ a single pair of anode-cathodes 43, 45 or more than two pairs of such electrodes.

The electrolysis cell 11 may comprise four BDD electrodes, e.g. 43, 45, with leads 53, 55. The spacing between the electrodes, e.g. 43, 45, may be in the range of 3-5 mm (y direction) and the grid-separator felt-grid stack should occupy no more than 3 mm. The separator felt material 47 need not extend beyond the electrodes 43, 45, which individually are, in an illustrative embodiment, 1 x 1 cm². In an illustrative embodiment, the thickness of the separator 47 may be between 0.5 and 1 mm, not including the support grids 48. The electrodes 43, 45 and separator felt 47 are aligned and distanced to specification, but the cell volume can be larger (extended in z and y dimensions) if the design calls for extra spacing to accommodate welding or fixturing features or pressure-flow characteristics. One embodiment supports laminar flow to avoid sedimentation resulting from turbulence-induced "no-flow" regions. No leaking should be permitted between the anode and cathode chambers beginning from the front end of the separator felt 47 and continuing downstream.

The support grid 48 can be polymer thin lanes directed parallel with flow (x direction). There should be no significant forces which push the separator 47 out of position and obviously the polymer support grid desirably takes up the least amount of area (xy) to avoid blockage of the ion path.

The BDD electrodes, e.g. 43, 45, which, in one embodiment may be 1 x 1 cm² (xy) and ~0.5-0.7 mm thick (z), are pressed into a trough 46 in the half-cell and connected to a titanium or copper lead 53, 55, using, for example, i.e. a conductive epoxy glue 57 - the glue serving the multiple purposes of holding the electrodes 53, 45 in place, ensuring electrical contact between each electrode 43, 45 and its respective lead 53, 55, preventing saltwater access to the contacts, and preventing saltwater from leaking through a lead exit. If the fit between the electrodes 43, 45 and the trough 46 is somewhat tight, two glue holes may be established for the gluing process at the point where an electrical current lead is exiting.

The cell 11, by nature, is symmetric in the plane of the separator felt 47 (xy) and, in one embodiment, could be constructed from two identical halves. In such an embodiment, assembly may consist of gluing the lead, e.g. 53, and electrode, e.g. 43, in the half cell and then joining the two half cells together while placing the separator 47 and support grids 48 between the halves and welding the seam for permanent pressure tightness and a corrosion proof joint. The entrance and exit to the cell 11 may comprise a straight tube connector 50 for entrance and a T/Y structure 49, 51 (Fig. 3) for exits. Suitable materials for the cell 11 may include, for example, PE (polyethylene), PP (polypropylene), EPDM (ethylene propylene diene monomer), PEX (cross linked polyethylene), PVC (polyvinyl chloride), Teflon (trademark name), PS (polysulfone), or PU (polyurethane). The cell 11 should further stand up to normal city water pressure conditions.

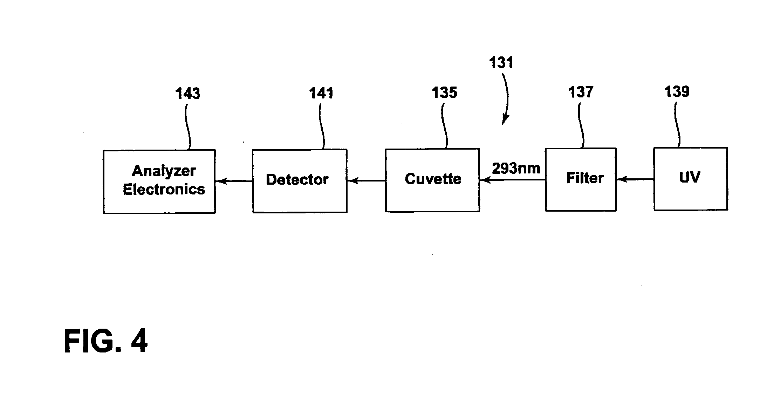

FIG. 4 depicts an illustrative spectrum analyzer 131. The analyzer 131 includes a UV source 139, a filter 137, a cuvette 135, a detector 141, and analysis electronics 143. The UV source 139 and filter 137 combination provide a 293 nm wavelength, which passes through the solution sample contained in the cuvette 135 and then to the detector 141, which may be a conventional UV detector, according to illustrative embodiments. The output of the detector 141 is applied to analysis electronics 143, which performs analog to digital (A-D) conversion. In one embodiment, analysis electronics 143 may comprise a suitable A-D converter and a microcontroller and/or computer. In one embodiment, suitable valving may be employed to control supply of successive solution samples to the cuvette 135. For example, in one embodiment, valve 60 (Fig. 3) may be used to provide samples of the salt water solution prior to and after electrolysis.

In one embodiment, the algorithms performed by the computing device 31 to determine chlorine content and pH are as follows:

A. Chlorine:

$$[Cl_2] = \frac{A_{293}^{alk} \cdot mw_{Cl_2} \cdot 1000}{\varepsilon \cdot l} \qquad \text{Equation (1)}$$

B. pH:

$$pH = 7.53 + \log \frac{A_{293}}{A_{293}^{alk} - A_{293}} \qquad \text{Equation (2)}$$

where $A_{293}$ is the absorption of ClO⁻ at or near 293 nm, $A_{293}^{alk}$ is the absorption at or near 293 nm of catholyte after the cathodic reaction has changed pH enough so that all chlorine is in hypochlorite form, "ε" is the extinction coefficient for hypochlorite, "$l$" is the length of the absorption path, $mw_{Cl2}$ is the molecular weight of chlorine. As noted above, the actual values used for $A_{293}$ and $A_{293}^{alk}$, may be determined based on a range around 293 nm, due to practical limitations of photodiodes used to generate a UV beam and of detectors employed to detect UV absorption.

The pH algorithm of equation (2) may be derived as follows:

$$[HClO] \overset{K}{\square} [H^+] + [ClO^-] \tag{3}$$

- reorganize:

$$K = \frac{[H^+][ClO^-]}{[HClO]}$$

$$\Updownarrow$$

$$pH = pK + \log \frac{[ClO^-]}{[HClO]} \tag{4, 5}$$

- and introduce the relations based on Lambert-Behr:

$$[ClO^-] \propto A_{293} \tag{6}$$

$$[HClO] + [ClO^-] \propto A_{293}^{alk} \tag{7}$$

$$A = [ClO^-]\varepsilon l \tag{8}$$

producing:

$$pH = 7.53 + \log \frac{A_{293}}{A_{293}^{alk} - A_{293}} \tag{9}$$

where $A_{293}$ is the absorption at or near 293 nm and $A_{293}^{alk}$ is the absorption at or near 293 nm of catholyte after cathodic reaction has changed pH enough so that all chlorine is in hypochlorite form. As is known, only hypochlorite absorbs UV at 293 nm.

Chlorine dissolved in water is hypochloric acid [HClO] with the corresponding base hypochlorite [ClO⁻].

$$Cl_2 \leftrightarrow HClO + Cl^- \leftrightarrow ClO^- + Cl^- \tag{10}$$

Therefore, producing or generating OH⁻ at the cathode of the cell 11 eliminates HClO and increases ClO⁻ and pH.

Studies indicate that the cathodic reaction is purely pH-changing, according to the reaction:

$$2H_2O + 2e^- \rightleftarrows H_2 + 2OH^- \qquad (11)$$

This reaction of course increases the alkalinity of the solution at the cathode. In one illustrative embodiment, the cathodic reaction is employed to change pH enough so that all chlorine $Cl_2$ in the solution is converted to hypochlorite ClO⁻ form. In one embodiment the pH is changed by electrolysis for such purpose to a level at or above 9.0.

Accordingly, in an illustrative method or process, the UV absorption $A_{293}$ of the salted spa water prior to electrolysis (e.g. at pH=7) is first measured by a spectrum analyzer, such as, for example, UV cell 15, then a second sample of the solution is subjected to electrolysis until the pH at the cathode is at or above 9.0. Once at a pH at or above 9.0, the UV absorption of the catholyte is measured by the spectrum analyzer to determine the UV absorption at 293 nm of the alkaline solution, which is denoted $A_{293}^{alk}$. The chlorine concentration $[Cl_2]$ and the pH of the solution are then determined, for example, by computing device 31, according to equations (1) and (2) above. In one embodiment, once $[Cl_2]$ and pH are determined, tests may be performed to determine whether the pH and $[Cl_2]$ values are within acceptable ranges and action thereafter taken to adjust those parameters. For example, if the values for chlorine and pH are outside of a range of 7<pH<8.5 and 3<ppm$[Cl_2]$<6ppm, where "ppm" means parts per million, corrective actions may be taken.

In one embodiment, the electrolysis process is performed at ∼100° F. The electrolysis process preferably should not consume power that leads to excessive cell heating.

In an illustrative embodiment, a microcontroller for computing pH and chlorine concentration and performing other control functions may comprise a PIC 32 microprocessor with 512K bytes of flash ROM, suitable amounts of RAM, EEPROM, and adequate I/O peripherals for various port functions. Additionally, for the purposes of this disclosure, a computer readable medium stores computer data, which data can include computer program code that is executable by a computer, in machine readable form. By way of example, and not limitation, a computer readable medium may comprise computer readable storage medium or media, for tangible or fixed storage of non-transitory data, or communication media for transient interpretation of code-containing signals. Computer readable storage medium or media, as used herein, refers to non-transitory physical or tangible storage (as opposed to signals) and includes without limitation volatile and non-volatile, removable and non-removable storage media implemented in any method or technology for the tangible storage of non-transitory information such as computer-readable instructions, data structures, program modules or other data. Computer readable storage media includes, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other physical or material medium which can be used to tangibly store the desired information or data or instructions and which can be accessed by a computer or processor. In certain embodiments, when suitable computer program code is loaded into and executed by a computer (including, e.g., a microprocessor, microcontroller, data processor, or similar computing device), the computer becomes a specially configured apparatus.

The invention also includes the following:

1. A method of measuring the level of chlorine in a first solution, the first solution comprising salt water, the method comprising:

measuring the UV absorption of a first sample of the first solution to generate a first absorption value;
subjecting the first solution to electrolysis to generate a catholyte;
measuring the UV absorption of said catholyte to generate a second absorption value; and
determining the level of chlorine in the first solution using the first and second absorption values.

2. The method of 1 wherein the pH of said catholyte is changed such that all chlorine in said catholyte is converted to ClO⁻ prior to measuring the absorption of the catholyte.
3. The method of 1 wherein the pH of said catholyte is adjusted to be at or above a selected level prior to measuring the absorption thereof.
4. The method of 3 wherein the selected pH level is 9.5 or higher.
5. The method of 1 wherein the level of chlorine in the first solution is determined using the equation:

$$[Cl_2] = \frac{A_{293}^{alk} \cdot mw_{Cl_2} \cdot 1000}{\varepsilon \cdot l}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form, "$\varepsilon$" is the extinction coefficient for hypochlorite, "$l$" is the length of the absorption path, and $mw_{Cl_2}$ is the molecular weight of chlorine.

6. A method of measuring the pH of a first solution, the first solution comprising salt water, the method comprising:

measuring the UV absorption of a first sample of the first solution to generate a first absorption value;
subjecting the first solution to electrolysis to generate a catholyte;
measuring the UV absorption of said catholyte to generate a second absorption value;
and
determining the pH of the first solution using the first and second absorption values.

7. The method of 6 wherein the pH of said catholyte is adjusted to be at or above a selected level prior to measuring the absorption thereof.
8. The method of 7 wherein the selected pH level is 9.5 or higher.
9. The method of 6 wherein the pH of the first solution is determined according to the equation:

$$pH = 7.53 + \log \frac{A_{293}}{A_{293}^{alk} - A_{293}}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form.

10. The method of 6 wherein the UV absorption of said first sample and said catholyte is measured at a wavelength in the range of 280 to 310 nanometers.
11. A method of measuring the chlorine level of a first solution, the first solution comprising salt water, the method comprising:

configuring an electrolysis cell to form separate anolyte and catholyte solutions from said first solution;
measuring the UV absorption of the first solution to generate a first absorption value;
employing electrolysis to adjust the pH of the catholyte solution to be above a selected threshold thereby forming a pH-adjusted catholyte solution;
measuring the UV absorption of pH adjusted catholyte solution to generate a second absorption value; and
determining the chlorine level of the first solution using said first and second absorption values.

12. The method of 11 wherein the selected threshold of pH level of the catholyte solution is 9.0 or higher.
13. The method of 11 wherein the level of chlorine in the first solution is determined using the equation:

$$[Cl_2] = \frac{A_{293}^{alk} \cdot mw_{Cl_2} \cdot 1000}{\varepsilon \cdot l}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form, "$\varepsilon$" is the extinction coefficient for hypochlorite, "$l$" is the length of the absorption path, $mw_{Cl2}$ is the molecular weight of chlorine.

14. The method of 12 wherein the level of chlorine in the first solution is determined using the equation:

$$[Cl_2] = \frac{A_{293}^{alk} \cdot mw_{Cl_2} \cdot 1000}{\varepsilon \cdot l}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form, "$\varepsilon$" is the extinction coefficient for hypochlorite, "$l$" is the length of the absorption path, and $mw_{Cl_2}$ is the molecular weight of chlorine.

15. The method of 11 wherein the UV absorption of said first sample and said catholyte is measured at a wavelength in the range of 280 to 310 nanometers.

16. The method of 14 wherein the UV absorption of said first sample and said catholyte is measured at a wavelength in the range of 280 to 310 nanometers.

17. The method of 11 further comprising employing said electrolysis cell to generate chlorine.

18. A method of measuring the pH level of a first solution, the first solution comprising salt water, the method comprising:

configuring an electrolysis cell to form separate anolyte and catholyte solutions from said first solution;
measuring the UV absorption of the first solution to generate a first absorption value;
employing electrolysis to adjust the pH of the catholyte solution to be above a selected threshold thereby forming a pH-adjusted catholyte solution;
measuring the UV absorption of pH-adjusted catholyte solution to generate a second absorption value; and
determining the pH level of the first solution using said first and second absorption values.

19. The method of 18 wherein the selected threshold of pH level of the catholyte solution is 9.0 or higher.

20. The method of 18 wherein the pH of the first solution is determined according to the equation:

$$pH = 7.53 + \log \frac{A_{293}}{A_{293}^{alk} - A_{293}}$$

where $A_{293}$ is the absorption of ClO⁻ at 293 nm or at a selected range around 293 nm, $A_{293}^{alk}$ is the absorption of the catholyte at 293 nm or at a selected range around 293 nm, after the electrolysis has changed the pH of the catholyte sufficiently so that all chlorine is in hypochlorite form.

21. The method of 19 wherein the pH of the first solution is determined according to the equation:

$$pH = 7.53 + \log \frac{A_{293}}{A_{293}^{alk} - A_{293}}$$

where $A_{293}$ is the absorption of ClO⁻ at 293 nm or at a selected range around 293 nm, $A_{293}^{alk}$ is the absorption of the catholyte at 293 nm or at a selected range around 293 nm, after the electrolysis has changed the pH of the catholyte sufficiently so that all chlorine is in hypochlorite form.

22. The method of 18 wherein the UV absorption of said first sample and said catholyte is measured at a wavelength in the range of 280 to 310 nanometers.

23. The method of 21 wherein the UV absorption of said first sample and said catholyte is measured at a wavelength in the range of 280 to 310 nanometers.

24. A tangible computer readable storage medium having non-transitory computer software or non-transitory program instructions stored thereon, which software or instructions when executed by one or more computing devices is operable to:

(a) receive a first UV absorption value representing the UV absorption of a first solution comprising salt water;
(b) receive a second UV absorption value representing the absorption value of a catholyte generated by electrolysis of said first solution; and
(c) determine the level of chlorine in the first solution using the first and second absorption values.

25. The computer readable storage medium of claim 24 wherein the level of chlorine in the first solution is determined using the equation:

$$[Cl_2] = \frac{A_{293}^{alk} \cdot mw_{Cl_2} \cdot 1000}{\varepsilon \cdot l}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form, "$\varepsilon$" is the extinction coefficient for hypochlorite, "$l$" is the length of the absorption path, and $mw_{Cl2}$ is the molecular weight of chlorine.

26. A tangible computer readable storage medium having non-transitory computer software or non-transitory program instructions stored thereon, which software or instructions when executed by one or more computing devices is operable to:

(a) receive a first UV absorption value representing the UV absorption of a first solution comprising salt water;
(b) receive a second UV absorption value representing the absorption value of a catholyte generated by electrolysis of said first solution; and
(c) determine the pH of the first solution using the first and second absorption values.

27. The computer readable storage medium of claim 26 wherein the pH of the first solution is determined according to the equation:

$$pH = 7.53 + \log \frac{A_{293}}{A_{293}^{alk} - A_{293}}$$

where $A_{293}$ is the absorption of ClO⁻ at 293 nm or at a selected range around 293 nm, $A_{293}^{alk}$ is the absorption of the catholyte at 293 nm or at a selected range around 293 nm, after the electrolysis has changed the pH of the catholyte sufficiently so that all chlorine is in hypochlorite form.

28. The method of 1 further comprising determining the pH of the first solution using said first and second absorption values.

29. The method of 28 wherein the pH of the first solution is determined according to the equation:

$$pH = 7.53 + \log \frac{A_{293}}{A_{293}^{alk} - A_{293}}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form.

30. The method of 11 further comprising determining the pH of the first solution using said first and second absorption values.

31. The method of 30 wherein the pH of the first solution is determined according to the equation:

$$pH = 7.53 + \log \frac{A_{293}}{A_{293}^{alk} - A_{293}}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form.

32. The method of 30 further comprising employing said electrolysis cell to generate chlorine.

Those skilled in the art will appreciate that various adaptations and modifications of the just described preferred embodiment can be configured without departing from the scope and spirit of the invention. Therefore, it is to be understood that, within the scope of the appended claims, the invention may be practiced other than as specifically described herein.

**Claims**

1. A method of measuring the level of chlorine in a first solution, the first solution comprising salt water, the method comprising:

   measuring the UV absorption of a first sample of the first solution to generate a first absorption value;
   subjecting the first solution to electrolysis to generate a catholyte;
   measuring the UV absorption of said catholyte to generate a second absorption value;
   and
   determining the level of chlorine in the first solution using the first and second absorption values.

2. The method of claim 1 wherein the pH of said catholyte is changed such that all chlorine in said catholyte is converted to ClO⁻ prior to measuring the absorption of the catholyte.

3. The method of claim 1 wherein the pH of said catholyte is adjusted to be at or above a selected level prior to measuring the absorption thereof.

4. The method of claim 3 wherein the selected pH level is 9.5 or higher.

5. The method of claim 1 wherein the level of chlorine in the first solution is determined using the equation:

$$[Cl_2] = \frac{A_{293}^{alk} \cdot mw_{Cl_2} \cdot 1000}{\varepsilon \cdot l}$$

where $A_{293}$ is the absorption of ClO⁻ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form, "$\varepsilon$" is the extinction coefficient for hypochlorite, "$l$" is the length of the absorption path, and $mw_{Cl2}$ is the molecular weight of chlorine.

6. A method of measuring the pH of a first solution, the first solution comprising salt water, the method comprising:

   measuring the UV absorption of a first sample of the first solution to generate a first absorption value;
   subjecting the first solution to electrolysis to generate a catholyte;
   measuring the UV absorption of said catholyte to generate a second absorption value;
   and
   determining the pH of the first solution using the first and second absorption values.

7. The method of claim 6 wherein the pH of said catholyte is adjusted to be at or above a selected level prior to measuring the absorption thereof.

8. The method of claim 7 wherein the selected pH level is 9.5 or higher.

9. The method of claim 6 wherein the pH of the first solution is determined according to the equation:

$$pH = 7.53 + \log \frac{A_{293}}{A_{293}^{alk} - A_{293}}$$

where $A_{293}$ is the absorption of ClO$^-$ at 293 nm or at a selected range around 293 nm, $A_{293}^{alk}$ is the absorption of the catholyte at 293 nm or at a selected range around 293 nm after the electrolysis has changed the pH of the catholyte sufficiently so that all chlorine is in hypochlorite form.

10. The method of claim 6 wherein the UV absorption of said first sample and said catholyte is measured at a wavelength in the range of 280 to 310 nanometers.

11. A method of measuring the chlorine level of a first solution, the first solution comprising salt water, the method comprising:

configuring an electrolysis cell to form separate anolyte and catholyte solutions from said first solution;
measuring the UV absorption of the first solution to generate a first absorption value;
employing electrolysis to adjust the pH of the catholyte solution to be above a selected threshold thereby forming a pH-adjusted catholyte solution;
measuring the UV absorption of pH adjusted catholyte solution to generate a second absorption value; and
determining the chlorine level of the first solution using said first and second absorption values.

12. A method of measuring the pH level of a first solution, the first solution comprising salt water, the method comprising:

configuring an electrolysis cell to form separate anolyte and catholyte solutions from said first solution;
measuring the UV absorption of the first solution to generate a first absorption value;
employing electrolysis to adjust the pH of the catholyte solution to be above a selected threshold thereby forming a pH-adjusted catholyte solution;
measuring the UV absorption of pH-adjusted catholyte solution to generate a second absorption value; and
determining the pH level of the first solution using said first and second absorption values.

13. A tangible computer readable storage medium having non-transitory computer software or non-transitory program instructions stored thereon, which software or instructions when executed by one or more computing devices is operable to:

(a) receive a first UV absorption value representing the UV absorption of a first solution comprising salt water;
(b) receive a second UV absorption value representing the absorption value of a catholyte generated by electrolysis of said first solution; and
(c) determine the level of chlorine in the first solution using the first and second absorption values.

14. A tangible computer readable storage medium having non-transitory computer software or non-transitory program instructions stored thereon, which software or instructions when executed by one or more computing devices is operable to:

(a) receive a first UV absorption value representing the UV absorption of a first solution comprising salt water;
(b) receive a second UV absorption value representing the absorption value of a catholyte generated by electrolysis of said first solution; and
(c) determine the pH of the first solution using the first and second absorption values.

FIG. 1

EP 2 853 888 A2

**FIG. 2**

**FIG. 3**

FIG. 4

EP 2 853 888 A2